# EUROPÄISCHE PATENTSCHRIFT

(11) **EP 0 822 843 B1**
(45) Veröffentlichungstag und Bekanntmachung des Hinweises auf die Patenterteilung: **13.11.2002**
(21) Anmeldenummer: 96914990.5
(22) Anmeldetag: 26.04.1996
(51) Int. Cl.: A61M 25/01

(54) **VORRICHTUNG ZUR VERBESSERTEN HANDHABUNG VON FÜHRUNGSDRÄHTEN INSBESONDERE BEI KATHETERUNTERSUCHUNGEN**
DEVICE FOR IMPROVING HANDLING OF GUIDING WIRES, IN PARTICULAR DURING EXAMINATIONS BY MEANS OF A CATHETER
DISPOSITIF FACILITANT LA MANIPULATION DE FILS DE GUIDAGE, NOTAMMENT LORS D'EXAMENS AU CATHETER

(30) Priorität: 28.04.1995 DE 19515761
(43) Veröffentlichungstag der Anmeldung: 11.02.1998
(73) Patentinhaber: Ischinger, Thomas, Prof. Dr., 81925 München (DE)
(72) Erfinder: Ischinger, Thomas, 81927 München (DE)
(74) Vertreter: Ruschke, Hans Edvard, Dipl.-Ing.
(86) Internationale Anmeldenummer: EP9601748
(87) Internationale Veröffentlichungsnummer: WO96033765

(56) Entgegenhaltungen:
- EP-A- 0 416 734
- WO-A-95/21566
- US-A- 4 616 652
- US-A- 4 799 496

## Beschreibung

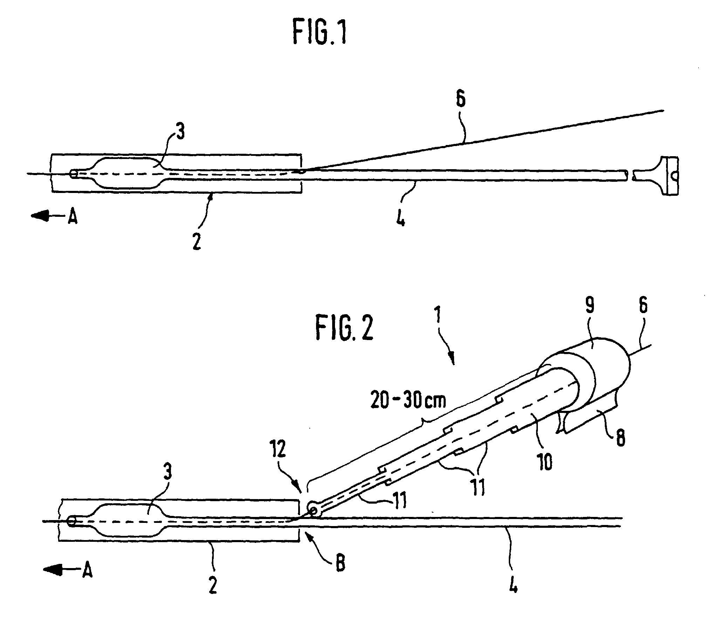

Bei der Ballondilatation oder Katheteruntersuchung beispielsweise im Herzen wird der Ballonkatheter oder ein anderer Katheter im allgemeinen in einem zuvor in den Patienten eingeführten Führungskatheter über einen sogenannten Führungsdraht vorgeschoben oder zurückgezogen. Bei der Monorail-Ausführung von Ballonkathetern verläßt der Führungsdraht das Drahtlumen des Ballonkatheters etwa 10 bis 30 cm proximal des Ballons (Abb. 1) und läuft dann außerhalb des Ballonkatheterschaftes aber parallel dazu innerhalb des Führungskatheters bis zu dessen proximalem Ende außerhalb des Patienten, so daß der Operateur am proximalen Ende des Führungskatheters den Schaft des Ballonkatheters und den Führungsdraht nebeneinander (bei Nicht-Monorail Systemen hintereinander) und separat manipulierbar in den Händen hält.

Im Verlauf einer Ballondilatation oder anderen Katheterisierungen kann es vorkommen, daß der Katheter ganz aus dem Führungskatheter zurückgezogen werden muß, etwa um gegen einen anderen ausgetauscht zu werden. Dabei ist es wichtig, daß der Führungsdraht trotz des Zurückziehens des (Ballon)Katheters an der einmal eingenommenen Stelle liegenbleibt, damit die vom Führungsdraht zuvor erreichte Stelle erreichbar bleibt. Das Zurückziehen des (Ballon)Katheters ist zunächst einmal völlig problemlos und wird einfach dadurch bewerkstelligt, daß der Führungsdraht mit der einen Hand festgehalten und der Schaft des (Ballon)Katheters mit der anderen Hand zurückgezogen wird, bis die Eintrittsöffnung des Führungsdrahtes in den Schaft des (Ballon)Katheters das proximale Ende des Führungskatheters erreicht hat. Allerdings bedarf es für die Endstrecke, in der der Führungsdraht innerhalb des Schaftes, z. B. des Ballonkatheters bei der Angioplastie, verläuft, einer besonderen Technik, damit der Draht nicht versehentlich mit dem (Ballon)Katheter zurückgezogen wird. Dies bedeutet, daß der Führungsdraht (wegen seiner geringen Knickstabilität) schrittweise in dem Maße gegen den (Ballon)Katheter vorgeschoben werden muß, wie das distale Ende des (Ballon)Katheters mit dem zentral darin verlaufenden Führungsdraht aus dem Führungskatheter herausgezogen wird. Diese Prozedur ist relativ umständlich, kostet Zeit und bedarf großer Aufmerksamkeit des Operateurs.

Eine Vorrichtung zur Handhabung von Führungsdrähten bei Katheteruntersuchungen ist z.B. im Dokument US-A-4 799 496 beschrieben und zeigt die im Oberbegriff des Anspruchs 1 definierten Merkmale.

Es ist daher die Aufgabe der vorliegenden Erfindung, eine Vorrichtung anzugeben, durch die das schwierige Rückzugsverfahren des Katheters über einen liegenden Führungsdraht vereinfacht wird.

Eine derartige Vorrichtung ist durch die Merkmale des Anspruches 1 gekennzeichnet.

Als Vorteil dieser erfindungsgemäßen Lösung ergibt sich, daß das Steuern des Drahtes durch den Operateur, also das vorschieben des Drahtes und zugleich das kontrollierte Drehen desselben, wesentlich erleichtert wird. Zugleich ist der Draht dadurch besser vom Katheterschaft zu unterscheiden, so daß beide leichter zu erkennen und zu hantieren sind, was im Interesse einer raschen und sicheren Behandlung von eminenter Wichtigkeit ist. Diese Vorrichtung ist in gleicher Weise für eine Vereinfachung und Verbesserung des Rückzuges von Kathetern mit koaxialem Drahtverlauf und sogenannten "Monorail"-Kathetern (Drahtverlauf vorzugsweise außerhalb des Katheterschaftes) bei verschiedensten Katheteruntersuchungen in den verschiedenen Organsystemen einzusetzen. Sie trägt auch dazu bei, eine einhändige Bedienung zu erleichtern bzw. erst zu ermöglichen.

Die erfindungsgemäße Vorrichtung umfaßt einen auf den Führungsdraht 6 aufschiebbaren Grundkörper mit einem vorzugsweise zentralen Lumen 10 für den Führungsdraht 6 und mit Festklemmitteln für denselben, wobei der Grundkörper 9 mit einer kanülenartigen distalen Verlängerung 11 versehen ist, die ggf. teleskopartig zusammenschiebbar ist. Das Maß, um das die kanülenartige Verlängerung teleskopartig zusammenschiebbar ist, entspricht z.B. beim Monorail-Katheter etwa der Länge, mit der der Führungsdraht am distalen Ende des Ballonkatheters zentral innerhalb des Ballonkatheterschaftes verläuft, entspricht also etwa der Strecke von der Eintrittsöffnung des Führungsdrahtes in den Ballonkatheterschaft bis zu seiner distalen Austrittsöffnung. Grundsätzlich kann aber in allen diagnostischen und therapeutischen Katheteranwendungen die Länge des Teleskopteiles je nach Anwendungsfall unterschiedlich sein. In seiner einfachsten Ausführungsform kann der Grundkörper zur kraftschlüssigen Festlegung am Führungsdraht mit einer zeichnerisch nicht dargestellten Rändelschraube oder dergleichen versehen sein, die gegen den durch das Zentrum des Grundkörpers 9 verlaufenden Führungsdraht 6 drückt und diesen gegen die gegenüberliegende Wandung klemmt. In einer anderen Ausführungsform kann diese Klemmung nach Art eines herkömmlichen Spannfutters wie bei Bohrmaschinen oder Drehbänken geschehen.

Die Figur 1 der Zeichnungen zeigt die herkömmliche Situation am Patienten, wobei man sich vorzustellen hat, daß sowohl der Führungskatheter 2 als auch der Führungsdraht 6 sehr viel länger als in der Zeichnung dargestellt sind und im Patienten liegen bzw. bis z. B. ins Herz reichen. Wie ersichtlich kann der Ballonkatheter in der in Figur 1 gegebenen Situation nicht weiter aus dem Führungskatheter 2 zurückgezogen werden, ohne zugleich auch den Führungsdraht 6 zurückzuziehen, weil letzterer im distalen Endbereich des Ballonkatheters innerhalb desselben verläuft (gestrichelt gezeichnet). Da der Führungsdraht die im Herzen oder einem anderen Organ (siehe Richtungspfeil A) erreichte Position trotz des weiteren Zurückziehens z.B. des Ballonkatheters 3 beibehalten soll, muß der Führungsdraht 6 also anläßlich des weiteren Zurückziehens des Ballonkatheters aus dem Führungskatheter 2 im gleichen Maße wie das Zurückziehen stattfindet, vorgeschoben werden. Dies ist ein schwieriger und leicht zu Fehlern führender Prozeß, den zu verbessern die vorliegende Erfindung sich zur Aufgabe gemacht hat. Dies wird erreicht z.B. durch eine aus der Figur 2 ersichtliche Ausführungsform der erfindungsgemäßen Vorrichtung 1, in die der Führungsdraht 6 vor dem Zurückziehen des (Ballon)Katheters 3, 4 eingelegt wird. Wie ersichtlich weist die Vorrichtung 1 in der gezeichneten Ausführungsform einen (langgestreckten) Grundkörper mit einer kanülenförmigen, teleskopartig in sich zusammenschiebbaren Verlängerung 11 auf, wobei der Führungsdraht 6 im Grundkörper 9 (in nicht näher dargestellter Weise) lösbar festgeklemmt werden kann, damit der Führungsdraht je nach Bedarf mit dem Grundkörper 9 in Axialund Umfangsrichtung festgelegt werden kann. Wenn letzteres der Fall ist, kann also der (Ballon)Katheter 3, 4 aus dem Führungskatheter 2 zurückgezogen werden, bis sich wieder die in Figur 2 dargestellte Situation ergibt, nämlich daß derjenige Punkt des Ballonkatheters erreicht wird, an dem der Führungsdraht 6 aus dem Ballonkatheter 3, 4 austritt (Austrittspunkt B) und beide zusammen aus dem Führungskatheter 2 austreten. Um nun den (Ballon)Katheter weiter aus dem Führungskatheter 2 herausziehen zu können, ohne daß sich der Führungsdraht 6 relativ zum zu behandelnden Organ verschiebt, wird die distale Verlängerung 11 oder Teleskopnase im gleichen Maße zusammengeschoben, wie das letzte Ende des (Ballon)Katheters aus dem Führungskatheter 2 weiter herausgezogen wird, so daß sich im Ergebnis keine Axialverschiebung des Führungsdrahtes 6 ergibt. Anschließend kann die Klemmung des Führungsdrahtes innerhalb des Grundkörpers 9 der erfindungsgemäßen Vorrichtung z. B. durch Hebeldruck aufgehoben werden und die Vorrichtung 1 kann vom Führungsdraht abgezogen oder auf ihm verschoben werden, wenn dies gewünscht wird. Insgesamt ergibt sich durch die neuartige Vorrichtung eine verbesserte und einfachere bzw. schnellere und sicherere Handhabung der gesamten Katheterisierung.

In einer in den Zeichnungen nicht dargestellten alternativen Ausführungsform ist das zur Drahtaufnahme vorgesehene "Lumen" der gesamten Vorrichtung einschließlich des Grundkörpers 9 seitlich geöffnet, und zwar in Gestalt eines längsverlaufenden geraden oder wellenförmigen oder spiralförmig verlaufenden Schlitzes. Dadurch wird erreicht, daß der Draht 6 nicht in das Lumen 10 in Axialrichtung eingefädelt werden muß sondern in den vorgenannten Schlitz seitlich eingelegt werden kann, was die Handhabung in bestimmten Anwendungsfällen erleichtern wird.

### Bezugszeichenliste

- 1: Vorrichtung "Torquer"
- 2: Führungskatheter
- 3: Ballonkatheter
- 4: Ballonkatheter-Shaft
- 5 6: Angioplastie-Führungsdraht
- 7 8: Tuchklemme
- 9: Grundkörper
- 10: zentrales Lumen
- 11: Verlängerung
- 12: Verdickung/Spitze
- A: Richtung zum Herzen oder dgl.
- B: Austrittspunkt

## Patentansprüche

1. Vorrichtung zur verbesserten Handhabung eines Katheter-Führungsdrahtes (6) an dessen proximalem Ende, bestehend aus
- einem Grundkörper (9) mit einem längsverlaufenden Drahtaufnahme-Lumen (10) zur Aufnahme des Führungsdrahtes (6),
- einem Festspannmittel zum kraftschlüssigen Festklemmen des Führungsdrahtes im längsverlaufenden Lumen,
**gekennzeichnet durch**
eine vom Grundkörper (9) an seinem distalen Ende vorstehende und relativ dazu längsverschiebliche kanülenartige Verlängerung (11), die sich als Verlängerung des längsverlaufenden Lumens (10) in Richtung desselben erstreckt.

2. Vorrichtung nach Anspruch 1, **dadurch gekennzeichnet, daß** die kanülenartige Verlängerung (11) in sich teleskopartig zusammenschiebbar ist.

3. Vorrichtung nach Anspruch 1 oder 2, **dadurch gekennzeichnet, daß** das Festspannmittel am proximalen Ende der Längserstreckung des Grundkörpers (9) angeordnet ist.

4. Vorrichtung nach Anspruch 1, 2 oder 3, **dadurch gekennzeichnet, daß** ein weiteres Festspannmittel am distalen Ende der Längserstreckung des Grundkörpers angeordnet ist.

5. Vorrichtung nach einem der voranstehenden Ansprüche, **dadurch gekennzeichnet, daß** das Festspannmittel durch einen am Grundkörper angeordneten Hebel betätigbar ist, dessen Betätigung das Festspannmittel in Eingriff mit dem Führungsdraht (6) bringt.

6. Vorrichtung nach einem der Ansprüche 1 bis 4, **dadurch gekennzeichnet, daß** das Festspannmittel durch einen am Grundkörper angeordneten Hebel betätigbar ist, dessen Betätigung das im Ruhezustand mit dem Führungsdraht verspannte Festspannmittel löst.

7. Vorrichtung nach einem der voranstehenden Ansprüche 5 und 6, **dadurch gekennzeichnet, daß** die Betätigung des Hebels zugleich die kanülenartige Verlängerung zur Längsverschiebung gegenüber dem Grundkörper freigibt.

8. Vorrichtung nach einem der voranstehenden Ansprüche, **dadurch gekennzeichnet, daß** außen am Grundkörper (9) eine Tuchklemme (8) zur Befestigung der Vorrichtung (1) an einem Abdecktuch vorgesehen ist.

9. Vorrichtung nach einem der voranstehenden Ansprüche, **dadurch gekennzeichnet, daß** die Spitze am distalen Ende der kanülenartigen Verlängerung olivenartig oder pilzförmig verdickt ist, um ein Eindringen in den Katheterschaft (4) zu vermeiden.

10. Vorrichtung nach dem voranstehenden Anspruch 9, **dadurch gekennzeichnet, daß** die verdickte Spitze (12) seitlich halbmondartig ausgespart ist zur Anpassung an den Schaft (4) des Ballonkatheters (3), also auf einer Längsseite zur besseren Anpassung eine Konkavität aufweist.

11. Vorrichtung nach einem der voranstehenden Ansprüche, **dadurch gekennzeichnet, daß** die kanülenartige Verlängerung (11) durch Federdruck ausfahrbar ist, z. B. durch eine mechanische Feder oder durch Gasdruck.

## Claims

1. Device for improving the handling of a catheter guide wire (6) at the proximal end thereof, consisting of
- a basic body (9) having a wire receiving lumen (10) extending longitu dinally therethrough fro receiving a guide wire(6),
- clamping means for the force-locked clamping of the guide wire inside the longitudinally extended lumen,
**characterized by**
- a cannula-like extension (11) on basic body (9), said extension projecting from basic body (9) at the distal end thereof and being longitudinally moveable relative thereto, said cannula-like extension extending as an extension of longitudinally extending lumen in the direction thereof.

2. Device as in claim 1, **characterized in that** cannula-like extension (11) is collapsible in telescope-like fashion.

3. Device as in claim 1 or 2, **characterized in that** said clamping means is provided at the proximal end of the longitudinal extend of the basic body (9).

4. Device as in claim 1, 2 or 3, **characterized by** an additional clamping means being provided at the distal end of the longitudinal extent of the basic body.

5. Device as in any one of the preceding claims, **characterized by** said clamping means being actuatable by a lever arm mounted on the basic body and, upon actuation, causing the clamping means to engage guide wire (6).

6. Device as in any one of claims 1 to 4, **characterized by** said clamping means being actuatable by a lever arm mounted on the basic body which, when actuated, releases the clamping means clamping the guide wire in the quiescent condition thereof.

7. Device as in one of claims 5 and 6, **characterized in that** actuation of said lever arm at the same time releases said cannula-like extension for longitudinal movement relative to the basic body.

8. Device as in any one of the preceding claims, **characterized in that** basic body (9) has exteriorly attached thereto a sheet clamp (8) to allow dev die (1) to be attached to a sheet or drape.

9. Device as in any one of the preceding claims, **characterized in that** the tip and the end of the cannula-like extension is expanded in olive or mushroom shape so as to prevent its entry into catheter shank (4).

10. Device as in claim 9, **characterized in that** said expanded tip (12) has a crescent-shaped lateral recess formed therein for conformance with the shank (4) of balloon catheter (3), i. e. it has a concave recess formed in a longitudinal side thereof for improved conformance.

11. Device as in any one of the preceding claims, **characterized by** cannula-like extension (11) being biased to extend by resilient pressure exerted by means such as a mechanical spring or gas pressure.

## Revendications

1. Dispositif pour faciliter la manipulation d'un fil de guidage de cathéter (6) à l'extrémité proximale de celui-ci, consistant en
- un corps de base (9) ayant une lumière de logement de fil longitudinale (10) pour le logement du fil de guidage (6),
- un moyen de serrage pour le blocage en force du fil de guidage dans la lumière longitudinale,
**caractérisé par** un prolongement en forme de canule (11), dépassant du corps de base (9) à son extrémité distale et déplaçable longitudinalement par rapport à celui-ci, et qui s'étend comme prolongement de la lumière longitudinale (10) dans la direction de celle-ci.

2. Dispositif selon la revendication 1, **caractérisé en ce que** le prolongement en forme de canule (11) est rétractable en lui-même de façon télescopique.

3. Dispositif selon la revendication 1 ou 2, **caractérisé en ce que** le moyen de serrage est disposé à l'extrémité proximale de l'extension longitudinale du corps de base (9).

4. Dispositif selon la revendication 1, 2 ou 3, **caractérisé en ce qu'**un autre . moyen de serrage est disposé à l'extrémité distale de l'extension longitudinale du corps de base.

5. Dispositif selon l'une des revendications précédentes, **caractérisé en ce que** le moyen de serrage peut être actionné par l'intermédiaire d'un levier disposé sur le corps de base, dont la manoeuvre amène le moyen de serrage en contact avec le fil de guidage (6).

6. Dispositif selon l'une des revendications 1 à 4, **caractérisé en ce que** le moyen de serrage peut être actionné par l'intermédiaire d'un levier disposé sur le corps de base, dont la manoeuvre détache le moyen de serrage arrimé au fil de guidage au repos.

7. Dispositif selon l'une des revendications précédentes 5 et 6, **caractérisé en ce que** la manoeuvre du levier libère directement le prolongement en forme de canule pour le déplacement longitudinal par rapport au corps de base.

8. Dispositif selon l'une des revendications précédentes, **caractérisé en ce qu'**une pince en tissu (8) est disposée à l'extérieur sur le corps de base (9) pour la fixation du dispositif (1) à un tissu de recouvrement.

9. Dispositif selon l'une des revendications précédentes, **caractérisé en ce que** la pointe à l'extrémité distale du prolongement en forme de canule est épaissie en forme d'olive ou de champignon, pour éviter qu'elle pénètre dans la tige du cathéter (4).

10. Dispositif selon la revendication précédente 9, **caractérisé en ce que** la pointe épaissie (12) est évidée latéralement en forme de demi-lune pour s'ajuster sur la tige (4) du cathéter à ballonnet (3), c'est-à-dire présente une concavité sur une face latérale pour un meilleur ajustement.

11. Dispositif selon l'une des revendications précédentes, **caractérisé en ce que** le prolongement en forme de canule (11) peut être sorti sous une pression de ressort, par exemple par l'intermédiaire d'un ressort mécanique ou par l'intermédiaire d'une pression de gaz.
